# EUROPEAN PATENT APPLICATION

(11) **EP 4 641 577 A1**
(43) Date of publication of application: **29.10.2025**
(21) Application number: 23906915.6
(22) Date of filing: 15.12.2023
(51) Int. Cl.: G16C 60/00

(54) **DESIGN EVALUATION DEVICE, DESIGN EVALUATION METHOD, AND PROGRAM**

(30) Priority: 23.12.2022 JP 2022207109
(71) Applicant: Resonac Corporation, Tokyo 105-7325 (JP)
(72) Inventor: LEE, Haein, Tokyo 105-7325 (JP); OGAWA, Masahiro, Tokyo 105-7325 (JP); TAKEMOTO, Shimpei, Tokyo 105-7325 (JP); OKUNO, Yoshishige, Tokyo 105-7325 (JP)
(74) Representative: Strehl & Partner mbB
(86) International application number: PCT/JP2023/044991
(87) International publication number: WO 2024/135553

(57) **Abstract**

A design evaluation device evaluates a degree of extrapolation of a candidate design of a target substance. The design evaluation device includes a reference design determination unit configured to determine a reference setting indicating a formulation of a target substance to be produced using two or more material substances, a candidate design determination unit configured to determine a candidate design indicating the formulation that is different from the reference design, a class determination unit configured to determine a class indicating a degree of extrapolation of the candidate design, based on a classification indicating a difference in the formulation between the candidate design and the reference design, and a result output unit configured to output the candidate design and the class in association with each other.

## Description

### TECHNICAL FIELD

The present disclosure relates to design evaluation devices, design evaluation methods, and programs.

### BACKGROUND ART

Conventionally, development of materials is based on the experience, intuition, or the like of engineers. In recent years, techniques for improving an efficiency of developing the materials by utilizing information processing techniques, such as machine learning or the like, are used.

For example, Patent Document 1 discloses a design support system for designing a new chemical product. The design support system disclosed in Patent Document 1 plots an existing point corresponding to a first parameter related to an existing chemical product and depletion points that become candidates of a second parameter for designing a new chemical product in a virtual space, and extracts a depletion point having a distance from the existing point greater than a specific distance.

### PRIOR ART DOCUMENTS

### PATENT DOCUMENTS

Patent Document 1: Japanese Laid-Open Patent Publication No. 2022-71456

### DISCLOSURE OF THE INVENTION

### PROBLEMS TO BE SOLVED BY THE INVENTION

However, there is a problem in that the conventional technique does not evaluate a degree of extrapolation of a candidate design for a target substance. When selecting material substances and formulations thereof from a prediction by the machine learning, a large error is likely to occur between a predicted value and a value obtained by experiment in an extrapolation region greatly separated from existing experimental data. For this reason, if the experiment is conducted based on the candidate design without understanding the degree of extrapolation of the candidate design, an expectation, a reliability, or the like of the design support system may deteriorate.

In view of the technical problem described above, it is an object of the present disclosure to evaluate the degree of extrapolation of the candidate design for the target substance.

### MEANS OF SOLVING THE PROBLEMS

The present disclosure includes the following configurations.
[1] A design evaluation device comprising:
   a reference design determination unit configured to determine a reference setting indicating a formulation of a target substance to be produced using two or more material substances;
   a candidate design determination unit configured to determine a candidate design indicating the formulation that is different from the reference design;
   a class determination unit configured to determine a class indicating a degree of extrapolation of the candidate design, based on a classification indicating a difference in the formulation between the candidate design and the reference design; and
   a result output unit configured to output the candidate design and the class in association with each other.
[2] The design evaluation device according to [1] above, wherein the class is preset with respect to a combination of a number of different types of the material substances and a number of different formulation amounts of the material substances.
[3] The design evaluation device according to [2] above, wherein the class is set with respect to the combination, in response to a user operation.
[4] The design evaluation device according to any one of [1] to [3] above, wherein:
   the class determination unit is configured to determine the class of each candidate design of a plurality of candidate designs, and
   the result output unit is configured to output a candidate design in which the class falls within a predetermined range among the plurality of candidate designs.
[5] The design evaluation device according to [4] above, wherein:
   the class determination unit is configured to further determine the class, based on a classification indicating a difference in the formulation between a candidate design of the plurality of candidate designs and an experimented design having a known physical property of the target substance for each candidate design among the plurality of candidate designs, and
   the result output unit is configured to output the candidate design in which both the class with respect to the reference design and the class with respect to the experimented design fall within the range.
[6] The design evaluation device according to [5] above, wherein the result output unit is configured to output information related to the reference design or the experimented design indicating the material substance having a formulation type or amount different from the candidate design.
[7] A design evaluation method by which a computer performs:
   a procedure determining a reference design indicating a formulation of a target substance to be produced using two or more material substances;
   a procedure determining a candidate design indicating the formulation that differs from the reference design;
   a procedure determining a class indicating a degree of extrapolation of the candidate design, based on a classification indicating a difference in the formulation between the candidate design and the reference design; and
   a procedure outputting the candidate design and the class in association with each other.
[8] A program for causing a computer to execute:
   a procedure determining a reference design indicating a formulation of a target substance to be produced using two or more material substances;
   a procedure determining a candidate design indicating the formulation that differs from the reference design;
   a procedure determining a class indicating a degree of extrapolation of the candidate design, based on a classification indicating a difference in the formulation between the candidate design and the reference design; and
   a procedure outputting the candidate design and the class in association with each other.

### EFFECTS OF THE INVENTION

According to one aspect of the present disclosure, it is possible to evaluate the degree of extrapolation of the candidate design for the target substance.

### BRIEF DESCRIPTION OF THE DRAWINGS

[FIG. 1] FIG. 1 is a block diagram illustrating an example of an overall configuration of a design support system.
[FIG. 2] FIG. 2 is a block diagram illustrating an example of a hardware configuration of a computer.
[FIG. 3] FIG. 3 is a block diagram illustrating an example of a functional configuration of the design support system.
[FIG. 4] FIG. 4 is a flow chart illustrating an example of a processing procedure of a design evaluation method.
[FIG. 5] FIG. 5 is a diagram illustrating examples of evaluation criteria.
[FIG. 6] FIG. 6 is a diagram illustrating examples of a newness class.
[FIG. 7] FIG. 7 is a diagram illustrating an example of an evaluation criterion input screen.
[FIG. 8] FIG. 8 is a diagram illustrating an example of a reference design input screen.
[FIG. 9] FIG. 9 is a diagram illustrating an example of a candidate design table.
[FIG. 10] FIG. 10 is a diagram illustrating an example of an extraction condition input screen.
[FIG. 11] FIG. 11 is a flow chart illustrating an example of a proposed design extraction process.
[FIG. 12] FIG. 12 is a diagram illustrating an example of an experimental result table.
[FIG. 13] FIG. 13 is a diagram illustrating an example of a result display screen.
[FIG. 14] FIG. 14 is a diagram illustrating an example of an evaluation result file.

### MODE OF CARRYING OUT THE INVENTION

Embodiments of the present invention will now be described with reference to the accompanying drawings. In the specification and the drawings, constituent elements having substantially the same functional configuration are designated by the same reference numerals, and a redundant description thereof will be omitted.

### [Embodiments]

An embodiment of the present disclosure relates to a design support system for supporting design of a substance that is produced using a plurality of substances. Hereinafter, a substance to be designed is referred to as a "target substance", and a substance used for producing the target substance is referred to as a "material substance".

Examples of the target substance in the present embodiment include resins, alloys, or the like. Examples of the material substance in the present embodiment include monomers, polymers, additives, or the like. The target substance and the material substance are not limited to the substances described above, and may be any substance produced using a plurality of substances.

Conventionally, development of materials is based on the experience, intuition, or the like of engineers. In recent years, techniques for improving an efficiency of developing the materials by utilizing information processing techniques, such as machine learning or the like, are used. On the other hand, when selecting materials or formulations thereof from a prediction result of the machine learning, a professional engineer needs to make a final determination. For this reason, it is important to provide information that serves as a criterion for making the determination on the prediction result of the machine learning.

When selecting the materials or the formulations thereof from the prediction result of the machine learning, a large error is likely to occur between a predicted value and a value obtained by experiment according to a design selected from an extrapolation region greatly separated from existing experimental data. If the experimental result based on the design provided by the design support system greatly differs from the prediction result, an expectation, a reliability, or the like of the design support system may deteriorate.

In addition, in the development of materials using the machine learning, various optimization methods, with focus on Bayesian optimization or the like, are being utilized. In these optimization methods, a design to be experimented on next is selected according to a predetermined criterion based mainly on the predicted value, the degree of extrapolation, or the like. However, the design selected by the machine learning may differ from a direction of the experiment desired by an experimenter or the like. The direction of the experiment includes not using (or reducing an amount used of) a specific material substance, adjusting a balance of a plurality of physical properties, or the like, for example. If the experimenter cannot reflect the desired direction of the experiment in the design support system, a motivation or the like to repeat the experiment may decline.

If an index obtained by quantifying the degree of extrapolation is provided in association with the prediction result provided by the design support system, a decrease in the deterioration of the expectation, reliability, or the like with respect to the design support system can be expected. In addition, if information indicating the direction of the experiment can be input to the design support system and a prediction result according to the direction of the experiment is provided, a decrease in the decline of the motivation or the like to repeat the experiment can be expected.

The degree of extrapolation is a degree indicating how far a region, in which a design to be evaluated (hereinafter also referred to as a "candidate design") is extrapolated, is separated from a design serving as a criterion for evaluation (hereinafter also referred to as a "reference design" or a "reference formulation"). The degree of extrapolation can also be regarded as an index indicating how similar the reference design and the candidate design are. The degree of extrapolation can also be interpreted as an indicator of how new the candidate design is (newness) with respect to the reference design, or how challenging it is to experiment with the candidate design (degree of challenge).

The formulation of the target substance includes discrete features (category variables) derived from the types of the material substances. For this reason, it is not appropriate to quantify the degree of extrapolation by a distance scale, such as the Euclidean distance or the like, generally used to evaluate a similarity. Although various distance scales have been proposed for mixed data in which continuous variables and categorical variables are mixed, it is difficult to determine a criterion for one calculated distance that enables all persons involved in the experiment or the like to understand and agree on the degree of extrapolation.

It is an object in one embodiment of the present disclosure to evaluate the degree of extrapolation of the candidate design for the target substance. In particular, one embodiment of the present disclosure aims to propose a candidate design that is highly convincing to those involved in the experiment or the like.

### <Overall Configuration of Design Support System>

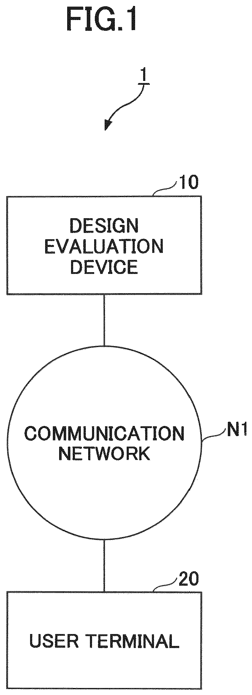

An overall configuration of the design support system according to the present embodiment will be described with reference to FIG. 1. FIG. 1 is a block diagram illustrating an example of the overall configuration of the design support system according to the present embodiment.

As illustrated in FIG. 1, a design support system 1 according to the present embodiment includes a design evaluation device 10 and a user terminal 20. The design evaluation device 10 and the user terminal 20 are communicably connected for data communication via a communication network N1, such as a local area network (LAN), the Internet, or the like.

The design evaluation device 10 refers to an information processing apparatus, such as a personal computer, a work station, a server, or the like that evaluates the degree of extrapolation of the candidate design indicating the formulation of the target substance, in response to a request from the user terminal 20. The design evaluation device 10 receives reference design data from the user terminal 20. The reference design data refers to data indicating a design (reference design) to be used as a criterion for evaluating the formulation of the target substance. The design evaluation device 10 transmits evaluation result data to the user terminal 20, based on the reference design data. The evaluation result data refers to data indicating a result of evaluating the degree of extrapolation for a design (candidate design) having a formulation different from the reference design.

The user terminal 20 refers to an information processing terminal, such as a personal computer, a tablet terminal, a smartphone, or the like operated by a user. The user terminal 20 receives the reference design, an evaluation criterion, and an extraction condition input by the user, and transmits the reference design data indicating the reference design, evaluation criterion data indicating the evaluation criterion, and extraction condition data indicating the extraction condition to the design evaluation device 10. The user terminal 20 receives the evaluation result data from the design evaluation device 10, and outputs the evaluation result indicated by the evaluation result data to a display device 506 or the like.

The overall configuration of the design support system 1 illustrated in FIG. 1 is merely an example, and various system configuration examples may be used depending on the use and purpose. For example, the design support system 1 may include one or more design evaluation devices 10 and/or one of more user terminals 20. For example, the design evaluation device 10 may be implemented by a plurality of computers or may be implemented as a service of a cloud computing system. Further, for example, the design support system 1 may be implemented by a stand-alone computer having functions to be provided in both the design evaluation device 10 and the user terminal 20. Classifications of the devices, such as the design evaluation device 10 and the user terminal 20 illustrated in FIG. 1, are merely an example.

### <Hardware Configuration of Design Support System>

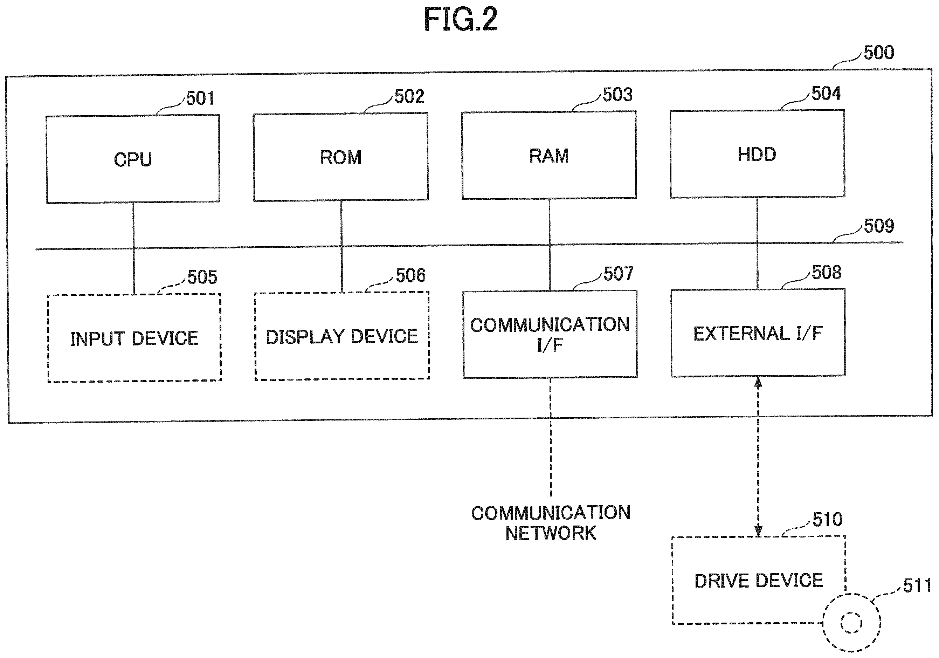

A hardware configuration of each device included in the design support system 1 according to the present embodiment will be described with reference to FIG. 2.

### <<Hardware Configuration of Computer>>

The design evaluation device 10 and the user terminal 20 according to the present embodiment are implemented by a computer, for example. FIG. 2 is a block diagram illustrating an example of the hardware configuration of a computer 500 in the present embodiment.

As illustrated in FIG. 2, the computer 500 includes a central processing unit (CPU) 501, a read only memory (ROM) 502, a random access memory (RAM) 503, a hard disk drive (HDD) 504, an input device 505, the display device 506, a communication interface (I/F) 507, and an external I/F 508. The CPU 501, the ROM 502, and the RAM 503 form a so-called computer. The hardware components of the computer 500 are connected to one another via a bus line 509. The input device 505 and the display device 506 may be used by being connected to the external I/F 508.

The CPU 501 is an arithmetic device configured to read programs and data from a storage device, such as the ROM 502, the HDD 504, or the like into the RAM 503, and to perform processes thereby implementing control and functions of the entire computer 500.

The ROM 502 is an example of a non-volatile semiconductor memory (storage device) that can retain programs and data even when the power is turned off. The ROM 502 functions as a main storage device configured to store various programs, data, or the like required by the CPU 501 to execute the various programs installed in the HDD 504. In particular, the ROM 502 stores a boot program, such as a basic input/output system (BIOS), an extensible firmware interface (EFI), or the like executed when booting the computer 500, and data, such as an operating system (OS) setting, a network setting, or the like.

The RAM 503 is an example of a volatile semiconductor memory (storage device) from which the programs and the data are erased when the power is turned off. The RAM 503 is a dynamic random access memory (DRAM), a static random access memory (SRAM), or the like, for example. The RAM 503 provides a work area to which the various programs are deployed when the CPU 501 executes the various programs installed in the HDD 504.

The HDD 504 is an example of a non-volatile storage device which stores the programs and the data. The programs and the data stored in the HDD 504 include the OS which is the basic software controlling the entire computer 500, and application programs for providing various functions on the OS, or the like. The computer 500 may utilize a storage device (for example, a solid state drive (SSD) or the like) using a flash memory as a storage medium, in place of the HDD 504.

The input device 505 is a touchscreen panel, operation keys or buttons, a keyboard, a mouse, or the like used by the user to input various signals, a microphone for inputting sound data such as voice, or the like.

The display device 506 is configured by a display, such as a liquid crystal display, an organic electroluminescent (EL) display, or the like for displaying a screen, and a speaker for outputting sound data such as voice, or the like.

The communication I/F 507 is an interface that is connected to the communication network and through which the computer 500 performs the data communication.

The external I/F 508 is an interface connected to an external device. The external device includes a drive device 510 or the like.

The drive device 510 is a device on which a recording medium 511 is set. The recording medium 511 includes a medium for optically, electrically, or magnetically recording information, such as a CD-ROM, a flexible disk, a magneto-optical disk, or the like. In addition, the recording medium 511 may include a semiconductor memory or the like for electrically recording information, such as a ROM, a flash memory, or the like. The computer 500 can read and/or write data from and/or to the recording medium 511 via the external I/F 508.

The various programs can be installed in the HDD 504 by setting the distributed recording medium 511 in the drive device 510 that is connected to the external I/F 508, and reading the various programs recorded in the recording medium 511 by the drive device 510, for example. Alternatively, the various programs can be installed in the HDD 504 by downloading the various programs from another network different from the communication network, via the communication I/F 507.

### <Functional Configuration of Design Support System>

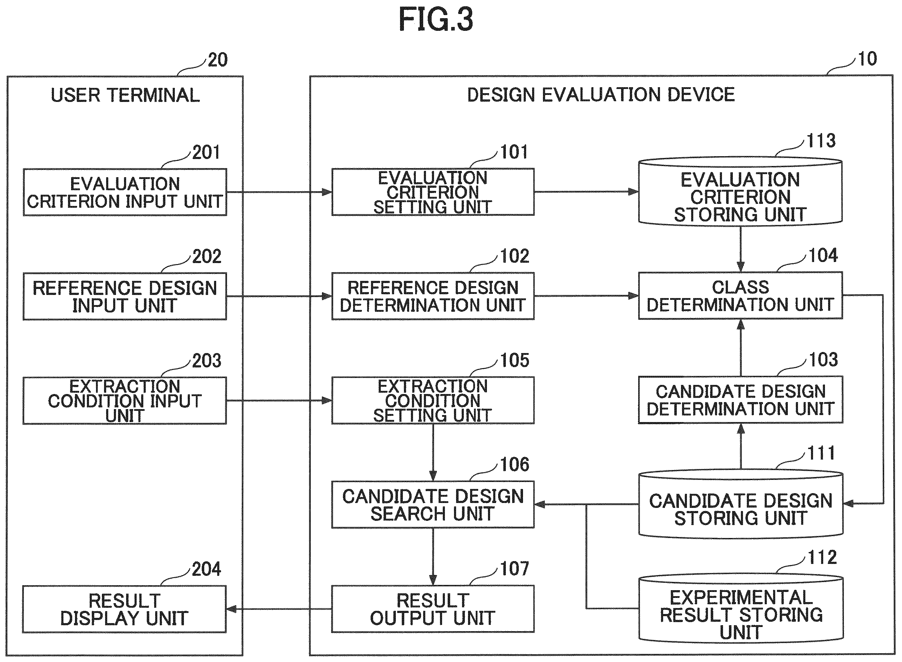

A functional configuration of the design support system according to the present embodiment will be described with reference to FIG. **3.** FIG. 3 is a block diagram illustrating an example of the functional configuration of the design support system 1 according to the present embodiment.

### <<Functional Configuration of Design Evaluation Device>>

As illustrated in FIG. 3, the design evaluation device 10 of the present embodiment includes an evaluation criterion setting unit 101, a reference design determination unit 102, a candidate design determination unit 103, a class determination unit 104, an extraction condition setting unit 105, a candidate design search unit 106, a result output unit 107, a candidate design storing unit 111, an experimental result storing unit 112, and an evaluation criterion storing unit 113.

The evaluation criterion setting unit 101, the reference design determination unit 102, the candidate design determination unit 103, the class determination unit 104, the extraction condition setting unit 105, the candidate design search unit 106, and the result output unit 107 are implemented when a program, deployed from the HDD 504 to the RAM 503 illustrated in FIG. 2, is executed by the CPU 501 and causes the CPU 501 to perform processes of these units. The candidate design storing unit 111, the experimental result storing unit 112, and the evaluation criterion storing unit 113 are implemented by the HDD 504 illustrated in FIG. 2.

The candidate design storing unit 111 prestores a plurality of candidate designs indicating formulations of mutually different target substances. The formulation of the target substance includes information indicating the type of the material substance, and information indicating a formulation amount of each material substance. The candidate designs are stored in a candidate design table constructed within the candidate design storing unit 111.

The information indicating the type of the material substance is identification information for identifying the type of the material substance. Examples of the identification information include an element name, an element number, a compound name, a chemical formula, or the like. The identification information is not limited to the above, and any information capable of identifying the material substance may be used. The information indicating the type of the material substance may include information indicating a category of the material substance. Examples of category of the material substance include resins, additives, fillers, or the like.

The information indicating the formulation amount of the material substance is information indicating an amount of the material substance per unit amount of the target substance. The formulation amount is expressed in parts per hundred (phr) or weight percent (wt%), for example. The expression method of the formulation amount is not limited to the above, and a generally used expression method may be used according to the type of the target substance or the like. In this embodiment, the formulation amount is expressed in parts per hundred (phr) for the sake of convenience.

The plurality of candidate designs are generated to have mutually different types of the material substances or mutually different formulation amounts of the material substances. The candidate design includes a prediction result of a physical property predicted by a trained model based on the candidate design (hereinafter also referred to as a "predicted characteristic value"). In the present embodiment, it is assumed that the plurality of candidate designs are randomly sampled by a comprehensive search using machine learning.

The experimental result storing unit 112 prestores a plurality of experimental results. The experimental results include an experimented design indicating a formulation in which the physical property of the target substance are known from past experiments, and an experimental result of the physical property obtained by the experiment based on the experimented design (hereinafter also referred to as "experimental characteristic value"). The experimental result is stored in an experimental result table constructed within the experimental result storing unit 112.

The evaluation criterion storing unit 113 stores the evaluation criterion used for evaluating the degree of extrapolation. The evaluation criterion stored in the evaluation criterion storing unit 113 is set by the evaluation criterion setting unit 101.

The evaluation criterion in the present embodiment refers to a criterion for determining a class indicating the degree of extrapolation (hereinafter also referred to as a "newness class" or simply as a "class"), based on the classification indicating a difference in the formulation amounts of the target substances. The classification in the present embodiment corresponds to a combination of the number of different types of material substances (hereinafter also referred to as a "formulation type distance" (TD; Type Distance)), and the number of different formulation amounts of material substances (hereinafter also referred to as a "formulation amount distance" (AD; Amount Distance)). The evaluation criterion in the present embodiment may be set for each type or category of the target substance.

The evaluation criterion setting unit 101 sets an evaluation criterion of the degree of extrapolation, based on evaluation criterion data received from the user terminal 20. The evaluation criterion setting unit 101 stores the evaluation criterion in the evaluation criterion storing unit 113.

The reference design determination unit 102 determines a reference design indicating the formulation of the target substance, based on reference design data received from the user terminal 20. The reference design refers to a design serving as a criterion for evaluating the degree of extrapolation.

The candidate design determination unit 103 determines a target design from a plurality of candidate designs stored in the candidate design storing unit 111. The target design refers to a candidate design to be evaluated of the degree of extrapolation.

The class determination unit 104 determines a newness class of the target design, based on the evaluation criterion read from the evaluation criterion storing unit 113. The class determination unit 104 classifies the difference in the formulation between the reference design determined by the reference design determination unit 102 and the target design determined by the candidate design determination unit 103, and determines the newness class of the target design according to the classification.

The extraction condition setting unit 105 sets an extraction condition for extracting a proposed design indicating the design to be proposed to the user, based on extraction condition data received from the user terminal 20. The extraction condition refers to a condition for extracting the proposed design from the target design (that is, the candidate design for which the newness class is determined) determined by the candidate design determination unit 103.

The extraction condition in the present embodiment includes a search range, a screening condition, and a constraint condition. The search range is a condition indicating the number of candidate designs to be included in the proposed design. The screening condition refers to a condition to be satisfied by the predicted characteristic value of the target substance. The constraint condition refers to a condition to be satisfied by the formulation amount of the material substance.

The candidate design search unit 106 extracts a proposed design from the plurality of candidate designs for which the newness class is determined by the class determination unit 104, based on the extraction condition set by the extraction condition setting unit 105. For example, the candidate design search unit 106 extracts the candidate designs in which the newness class falls within a predetermined range. In addition, the candidate design search unit 106 extracts a predetermined number of proposed designs for each newness class, for example.

Moreover, the candidate design determination unit 103 may extract only the candidate design including the predicted characteristic value satisfying the screening condition, for example. Further, the candidate design determination unit 103 may extract only the candidate design including the formulation amount of the material substance satisfying the restraint condition, for example.

The result output unit 107 transmits evaluation result data indicating the evaluation result of the degree of extrapolation to the user terminal 20. The evaluation result data includes the proposed design extracted by the candidate design search unit 106, and the newness class determined by the class determination unit 104 for the proposed design. The evaluation result data may include information related to a reference design or an experimented design indicating a material substance having a different type or a different formulation amount from the proposed design.

### <<Functional Configuration of User Terminal 20>>

As illustrated in FIG. 3, the user terminal 20 of the present embodiment includes an evaluation criterion input unit 201, a reference design input unit 202, an extraction condition input unit 203, and a result display unit 204.

The evaluation criterion input unit 201, the reference design input unit 202, and the extraction condition input unit 203 are implemented when a program, deployed from the HDD 504 to the RAM 503 illustrated in FIG. 2, is executed by the CPU 501 and the input device 505 and causes the CPU 501 and the input device 505 to perform processes of these units. The result display unit 204 is implemented when a program, deployed from the HDD 504 to the RAM 503 illustrated in FIG. 2, is executed by the CPU 501 and the display device 506 and causes the CPU 501 and the display device 506 to perform processes of these units.

The evaluation criterion input unit 201 receives an input of an evaluation criterion in response to a user operation. The evaluation criterion input unit 201 transmits evaluation criterion data indicating the evaluation criterion to the design evaluation device 10.

The reference design input unit 202 receives an input of a reference design in response to a user operation. The reference design input unit 202 transmits reference design data indicating the reference design to the design evaluation device 10.

The extraction condition input unit 203 receives an input of an extraction condition in response to a user operation. The extraction condition input unit 203 transmits extraction condition data indicating the extraction condition to the design evaluation device 10.

The result display unit 204 receives the evaluation result data from the design evaluation device 10. The result display unit 204 outputs the evaluation result indicated by the evaluation result data to the display device 506.

### <<Processing Procedure of Design Support System>

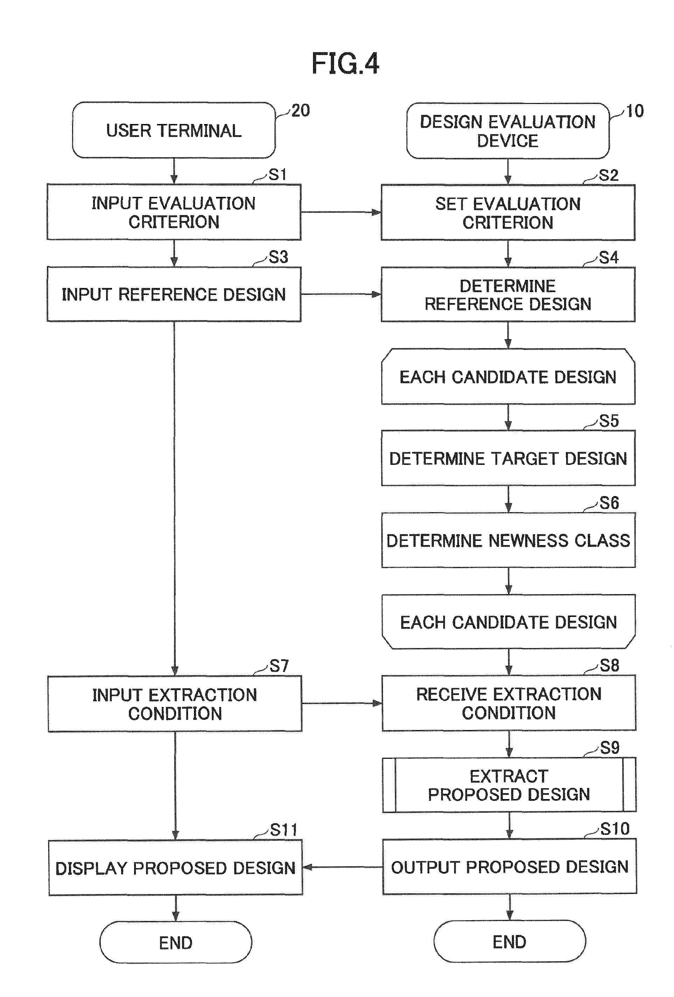

A processing procedure of a design evaluation method performed by the design support system 1 according to the present embodiment will be described with reference to FIG. 4 through FIG. 14. FIG. 4 is a flow chart illustrating an example of the processing procedure of the design evaluation method according to the present embodiment.

In step S1, the evaluation criterion input unit 201 of the user terminal 20 displays an evaluation criterion input screen for inputting an evaluation criterion on the display device 506. Next, the evaluation criterion input unit 201 receives the evaluation criterion input from the evaluation criterion input screen in response to a user operation. Subsequently, the evaluation criterion input unit 201 generates evaluation criterion data indicating the received evaluation criterion. Then, the evaluation criterion input unit 201 transmits the generated evaluation criterion data to the design evaluation device 10.

### <<Evaluation Criterion>>

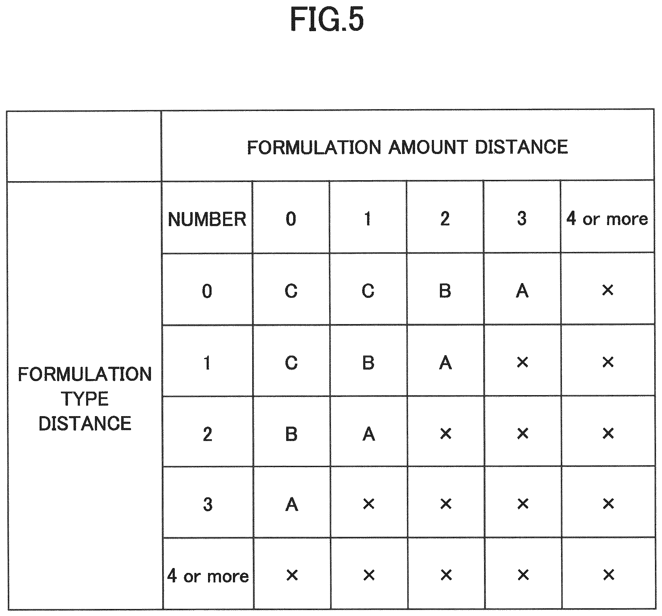

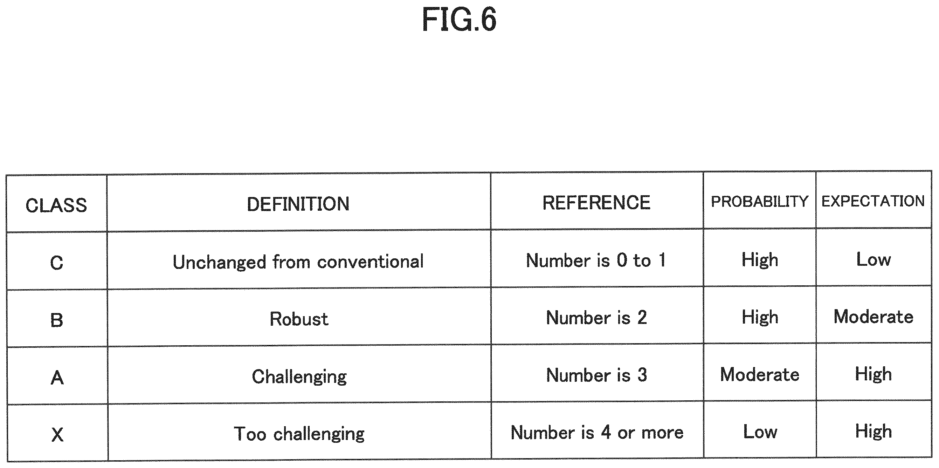

The evaluation criterion in the present embodiment will be described with reference to FIG. 5 and FIG. 6. FIG. 5 is a diagram illustrating examples of evaluation criteria in the present embodiment. FIG. 6 is a diagram illustrating an example of the newness class in the present embodiment.

As illustrated in FIG. 5, the evaluation criterion in the present embodiment is a matrix consisting of a number of different types of material substances (formulation type distance) and a number of different formulation amounts of the material substances (formulation amount distance). In the present embodiment, a row of the evaluation criterion corresponds to the formulation amount distance, and a column of the evaluation criterion corresponds to the formulation type distance, but the row and the column may be switched. Each element of the matrix corresponds to the combination of the formulation amount distance and the formulation type distance. The newness class is set for each element of the matrix.

The evaluation criterion in the present embodiment may be set for each type or each category of the target substance. In a case where the evaluation criterion is set for each type or each category of the target substance, the matrix indicating the evaluation criterion illustrated in FIG. 5 may be defined for each type or each category.

As illustrated in FIG. 6, the newness class in this embodiment include four kinds of classes defined by A, B, C, and X. However, the newness class can be designed arbitrarily according to a condition or the like for setting the number of classes, a symbol indicating each class, and a definition of each class.

The newness class A defines a challenging design. According to the newness class A, there is a moderate probability of obtaining an experimental result similar to the prediction result, but there is a high expectation value that the design is a new design different from the conventional design. The newness class A is set when a sum of the number of different types of material substances and the number of different formulation amounts of the material substances is 3.

The newness class B defines a robust design. According to the newness class B, there is a high probability of obtaining an experimental result similar to the prediction result, but there is a moderate expectation value that the design is a new design different from the conventional design. The newness class B is set when the sum of the number of different types of material substances and the number of different formulation amounts of the material substances is 2.

The newness class C defines a design unchanged from the conventional design. According to the newness class C, there is a high probability of obtaining an experimental result similar to the prediction result, but there is a low expectation value that the design is a new design different from the conventional design. The newness class C is set when the sum of the number of different types of material substances and the number of different formulation amounts of the material substances is 1 or less.

The newness class X defines a design that is too challenging. According to the newness class X, there is a low probability of obtaining an experimental result similar to the prediction result, but there is a high expectation value that the design is a new design different from the conventional design. The newness class X is set when the sum of the number of different types of material substances and the number of different formulation amounts of the material substances is 4 or greater.

### <<Evaluation Criterion Input Screen>>

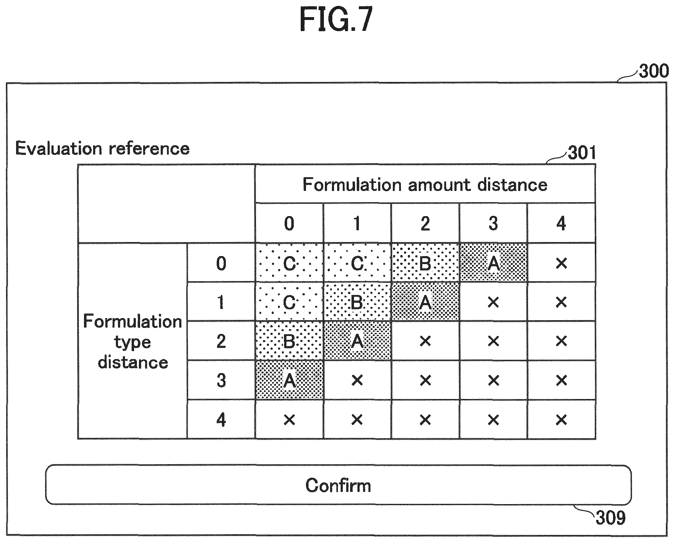

The evaluation criterion input screen according to the present embodiment will be described with reference to FIG. 7. FIG. 7 is a diagram illustrating an example of the evaluation criterion input screen according to the present embodiment.

As illustrated in FIG. 7, an evaluation criterion input screen 300 according to the present embodiment includes an evaluation criterion input field 301 and a confirm button 309.

The evaluation criterion input field 301 displays the evaluation criterion in an editable manner. The evaluation criterion input field 301 displays a matrix including the formulation type distance and the formulation amount distance. In each element of the matrix, a newness class corresponding to a combination of the formulation type distance and the formulation amount distance is displayed in a modifiable manner. The evaluation criterion input field 301 may display each element of the matrix in a color according to the newness class.

When the user inputs the evaluation criterion to the evaluation criterion input field 301 and presses the confirm button 309, the evaluation criterion input unit 201 receives the evaluation criterion input to the evaluation criterion input field 301.

A description will be made by referring back to FIG. 4. In step S2, the criterion setting unit 101 of the design evaluation device 10 receives the evaluation criterion data from the user terminal 20. Next, the evaluation criterion setting unit 101 acquires the evaluation criterion from the received evaluation criterion data. Subsequently, the evaluation criterion setting unit 101 stores the acquired evaluation criterion in the evaluation criterion storing unit 113.

In step S3, the reference design input unit 202 of the user terminal 20 displays the reference design input screen for inputting the reference design on the display device 506. Next, the reference design input unit 202 receives the reference design input from the reference design input screen in response to a user operation. Subsequently, the reference design input unit 202 generates the reference design data indicating the received reference design. Then, the reference design input unit 202 transmits the generated reference design data to the design evaluation device 10.

### <<Reference Design Input Screen>>

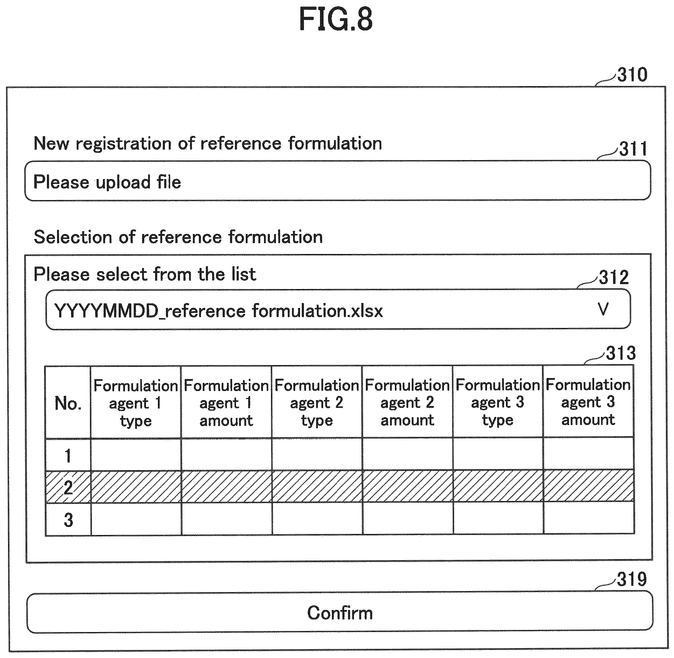

The reference design input screen in the present embodiment will be described with reference to FIG. 8. FIG. 8 is a diagram illustrating an example of the reference design input screen in the present embodiment.

As illustrated in FIG. 8, a reference design input screen 310 includes a file input field 311, a file selection field 312, a design selection field 313, and a confirm button 319.

The file input field 311 refers to an input field for inputting an electronic file (hereinafter also referred to as a "reference design file") in which the formulation of the target substance is described. The reference design file may include a description of a plurality of formulations. When the user drags and drops the reference design file to the file input field 311, the reference design file is uploaded to the design evaluation device 10. The reference design file may be uploaded by the user referring to a storage folder and selecting the reference design file.

The file selection field 312 displays the reference design file uploaded to the design evaluation device 10 in a selectable manner. When the user selects the reference design file in the file selection field 312, the formulation described in the reference design file is displayed in the design selection field 313.

The design selection field 313 displays reference designs described in the reference design file selected in the file selection field 312 in a selectable manner. In the present embodiment, the design selection field 313 displays one or more formulations in a table format, and when the user clicks a row corresponding to a desired formulation, the formulation corresponding to the row is selected.

When the user selects the formulation in the design selection field 313 and presses the confirm button 319, the reference design input unit 202 receives the formulation selected in the design selection field 313 as a reference design.

A description will be made by referring back to FIG. 4. In step S4, the reference design determination unit 102 of the design evaluation device 10 receives the reference design data from the user terminals 20. Next, the reference design determination unit 102 determines the reference design, based on the received reference design data. Subsequently, the reference design determination unit 102 transmits the determined reference design to the class determination unit 104.

In step S5, the candidate design determination unit 103 of the design evaluation device 10 reads the candidate designs from the candidate design table stored in the candidate design storing unit 111. Next, the candidate design determination unit 103 determines a target design from the read candidate designs. The candidate design determination unit 103 may select one target design in an order according to a predetermined rule or may select one target design at random. Subsequently, the candidate design determination unit 103 transmits the determined target design to the class determination unit 104.

### <<Candidate Design Table>>

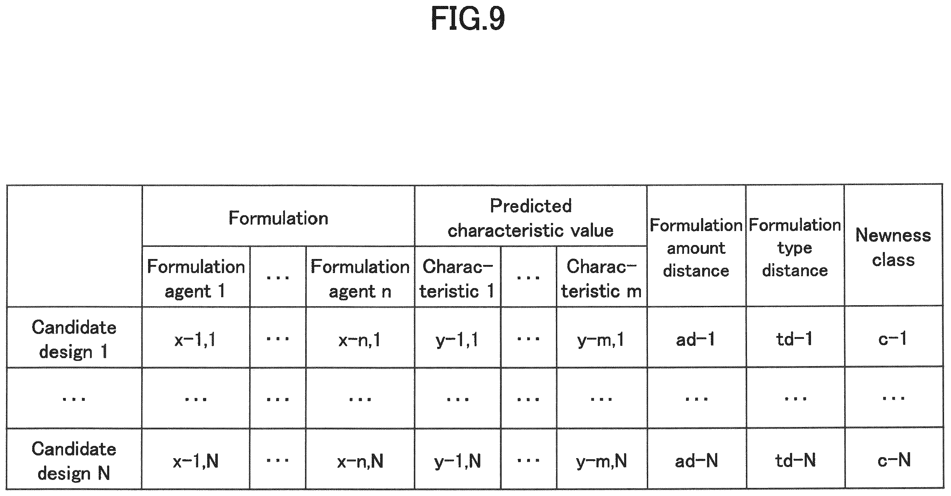

The candidate design table in the present embodiment will be described with reference to FIG. 9. FIG. 9 is a diagram illustrating an example of the candidate design table according to the present embodiment.

As illustrated in the FIG. 9, the candidate design table in the present embodiment has data items including formulation type 1, **...,** formulation type n (formulation), characteristic 1, ..., characteristic m (predicted characteristic value), formulation type distance, formulation amount distance, newness class, or the like.

The formulation refers to the formulation amount of each of n formulation types (formulation type 1, ..., formulation type n) that may be the material substance of the target substance. In this case, n is an integer greater than or equal to 2, and differs depending on the target substance.

The predicted characteristic value refers to m physical properties (characteristic 1, ..., characteristic m) predicted by the trained model based on the candidate design. In this case, m is an integer greater than or equal to 1, and differs depending on the type and category of the target substance.

The formulation type distance and the formulation amount distance refer to a formulation type distance and a formulation amount distance between the candidate design and the reference design, respectively. The newness class refers to a newness class of the candidate design with respect to the reference design. In an initial state, the formulation type distance, the formulation amount distance, and the newness class are not set. The formulation type distance, the formulation amount distance, and the newness class are set by the class determination unit 104 when determining the newness class of the candidate design.

A description will be made by referring back to FIG. 4. In step S6, the class determination unit 104 of the design evaluation device 10 receives the reference design from the reference design determination unit 102. In addition, the class determination unit 104 receives the target design from the candidate design determination unit 103. Next, the class determination unit 104 obtains a difference in the formulation between the reference design and the target design.

The difference in the formulation in this embodiment includes a formulation type distance and a formulation amount distance. The formulation type distance refers to the number of material substances that are not included in the plurality of material substances included in the reference design, among the plurality of material substances included in the target design. The formulation amount distance refers to the number of material substances for which the difference between the formulation amount of the reference design and the formulation amount of the target design is greater than or equal to a threshold value, among the material substances included in both the reference design and the target design. In the present embodiment, the threshold value is set to 10 phr, for example. However, the threshold value may be set to a different value according to the type or category of the material substance.

Subsequently, the class determination unit 104 reads the evaluation criterion from the evaluation criterion storing unit 113. Next, the class determination unit 104 classifies the difference in the formulations based on the evaluation criterion. The classification of the difference in the formulations corresponds to a combination of the formulation type distance and the formulation amount distance. For example, in the case of the evaluation criteria illustrated in FIG. 5, the classification of the difference in the formulations corresponds to each element of the matrix composed of the formulation amount distance and the formulation type distance.

Subsequently the class determination unit 104 determines the newness class according to the classification of the difference in the formulations based on the evaluation criterion. In the evaluation criteria illustrated in FIG. 5, the newness class is defined by each element of the matrix composed of the formulation type distance and the formulation amount distance. Accordingly, the class determination unit 104 determines the newness class defined by the element corresponding to the classification of the difference in the formulations in the evaluation criterion, as the newness class of the target design.

The class determination unit 104 stores the formulation type distance, the formulation amount distance, and the newness class in the candidate design table stored in the candidate design storing unit 111. Thus, the formulation type distance, the formulation amount distance, and the newness class are associated with the candidate design in the candidate design table.

The design evaluation device 10 repeatedly performs the processes of step S5 and step S6 until there is no candidate design selected as the target design by the candidate design determination unit 103. Hence, the newness class is determined for all of the candidate designs stored in the candidate design storing unit 111.

In step S7, the extraction condition input unit 203 of the user terminal 20 displays an extraction condition input screen for inputting an extraction condition on the display device 506. Next, the extraction condition input unit 203 receives the extraction condition input to the extraction condition input screen, in response to a user operation. Subsequently, the extraction condition input unit 203 generates extraction condition data indicating the received extraction condition. Then, the extraction condition input unit 203 transmits the generated extraction condition data to the design evaluation device 10.

The extraction condition input unit 203 may provide a status of the number of newness classes or the like at this point in time. In addition, the extraction condition input unit 203 may display the evaluation criterion input screen 300 illustrated in FIG. 7 on the display device 506, to enable editing of the evaluation criterion.

### <<Extraction Condition Input Screen>>

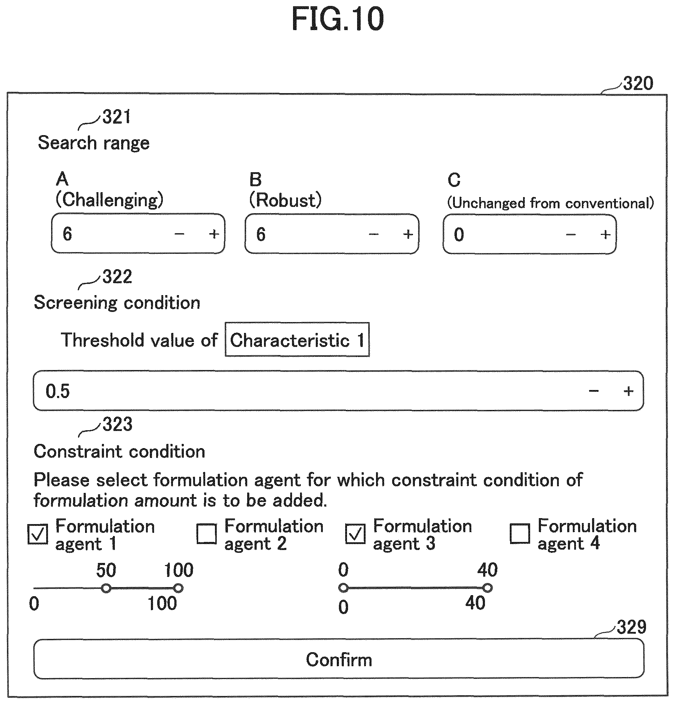

The extraction condition input screen in the present embodiment will be described with reference to FIG. 10. FIG. 10 is a diagram illustrating an example of the extraction condition input screen in the present embodiment.

As illustrated in FIG. 10, an extraction condition input screen 320 in the present embodiment includes a search range input field 321, a screening condition input field 322, a constraint condition input field 323, and a confirm button 329.

The search range input field 321 refers to an input field for inputting a search range. In this embodiment, the search range indicates information for setting the number of candidate designs to be extracted for each newness class. In the example illustrated in FIG. 10, the search range for extracting six candidate designs of the newness class A, extracting six candidate designs of the newness class B, and outputting twelve candidate designs including these extracted candidate designs, is input.

The screening condition input field 322 refers to an input field for inputting a screening condition. In the present embodiment, the screening condition is the condition to be satisfied by the predicted characteristic value based on the candidate design. In the example illustrated in FIG. 10, the screening condition for extracting the candidate design having the characteristic 1 of 0.5 or more is input.

The constraint condition input field 323 refers to an input field for inputting a constraint condition. In the present embodiment, the constraint condition is the condition to be satisfied by the formulation amount of the material substance. In the example illustrated in FIG. 10, the constraint condition for extracting the candidate design in which the formulation amount of the formulation type 1 is 50 phr to 100 phr and the formulation amount of the formulation type 3 is 0 to 40 phr is input.

The search range is a mandatory input item. The screening condition and the constraint condition are arbitrary input items. The user can arbitrarily determine whether or not to use the screening condition and the constraint condition.

When the user inputs at least the search range from the extraction condition input screen 320 and presses the confirm button 329, the extraction condition input unit 203 receives the extraction condition input from the extraction condition input screen 320.

A description will be made by referring back to FIG. 4. In step S8, the extraction condition setting unit 105 of the design evaluation device 10 receives the extraction condition from the user terminals 20. Next, the extraction condition setting unit 105 acquires the extraction condition from the received extraction condition data. Subsequently, the extraction condition setting unit 105 transmits the acquired extraction condition to the candidate design search unit 106.

In step S9, the candidate design search unit 106 of the design evaluation device 10 receives the extraction condition from the extraction condition setting unit 105. Next, the candidate design search unit 106 reads the candidate design associated with the newness class from the candidate design table stored in the candidate design storing unit 111. Subsequently, the candidate design search unit 106 extracts a proposed design from the candidate designs associated with the newness class, based on the extraction condition. Then, the candidate design search unit 106 transmits the extracted proposed design to the result output unit 107.

In a case where a screening condition is included in the extraction condition, the candidate design search unit 106 extracts a candidate design that matches the screening condition. In a case where the extraction condition includes a constraint condition, the candidate design search unit 106 extracts a candidate design that matches the constraint condition. Further, the candidate design search unit 106 extracts the proposed design from the candidate designs so as to satisfy the number for each newness class specified in the search range included in the extraction condition.

### <<Proposed Design Extraction Process>>

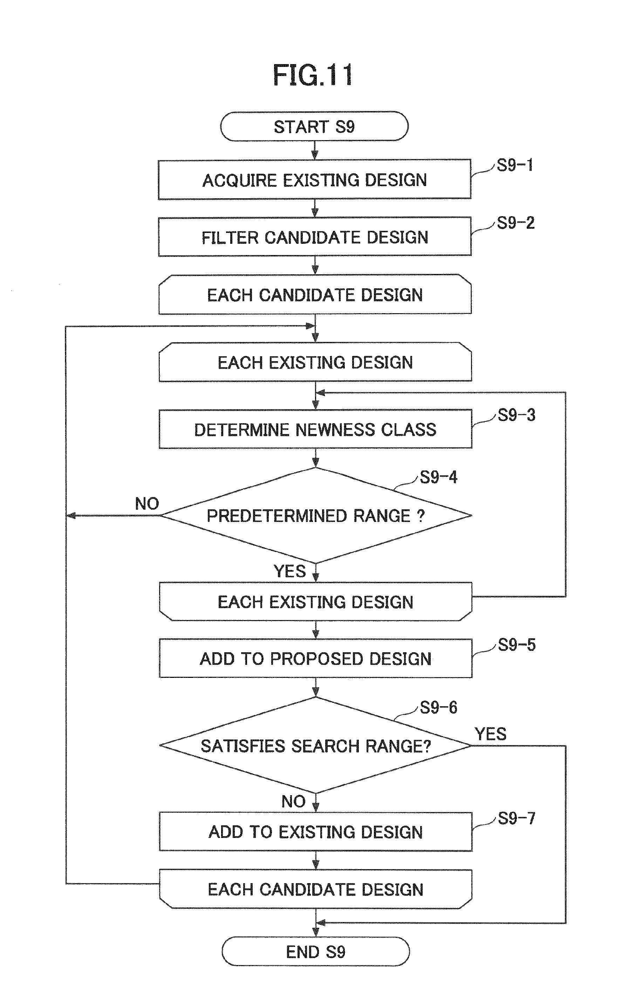

A proposed design extraction process (step S9 in FIG. 4) in the present embodiment will be described in more detail with reference to FIG. 11. FIG. 11 is a flow chart illustrating an example of the proposed design extraction process in the present embodiment.

In step S9-1, the candidate design search unit 106 reads an experimented design from the experimental result storing unit 112. Next, the candidate design search unit 106 adds the read experimented design to the existing design. The existing design is a set including the experimented design and the proposed design. In the initial state, the existing design includes only the experimented design. Each time a candidate design is added to the proposed design, the candidate design is added to the existing design, and finally, all of the experimented designs and the proposed design are included in the existing design.

When the proposed design is extracted based solely on the degree of extrapolation with respect to the reference design, a design similar to the experimented design, and a plurality of mutually similar candidate designs may be included in the proposed design. By including, in the proposed design, only the candidate design of the newness class within the predetermined range with respect to the existing design, it is possible to avoid the design similar to the experimented design or the plurality of mutually similar candidate designs from becoming included in the proposed design.

### <<Experimental Result Table>>

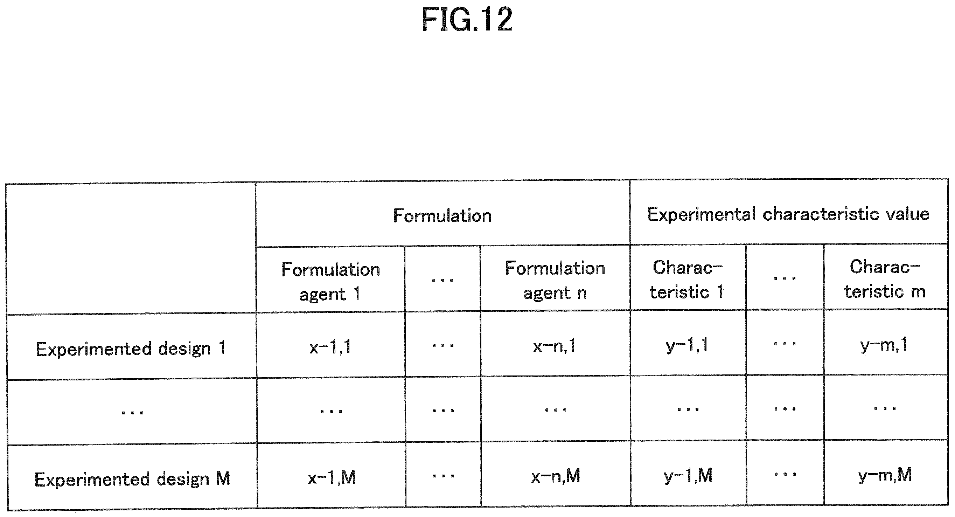

The experimental result table in the present embodiment will be described with reference to FIG. 12. FIG. 12 is a diagram illustrating an example of the experimental result table in the present embodiment.

As illustrated in FIG. 12, the experimental result table in the present embodiment has data items including formulation type 1, ..., formulation type n (formulation), and formulation type 1, ..., formulation type m (experimental property value).

The formulation is similar to the formulation in the candidate design table. The experimental property values are m physical properties (characteristic 1, ..., characteristic m) measured by an experiment based on the candidate design.

A description will be made by referring back to FIG. 11. In step S9-2, the candidate design search unit 106 reads a candidate design associated with the newness class from the candidate design storing unit 111. Next, the candidate design search unit 106 extracts a candidate design of the newness class in which the number of candidate designs in the search range is 1 or greater. That is, the candidate design of the newness class X, and the candidate design of the newness class in which the number of candidate designs in the search range is 0, are discarded. Subsequently, the candidate design search unit 106 performs a filtering of the candidate designs, based on the screening condition and the constraint condition. That is, the candidate design search unit 106 extracts a candidate design satisfying both the screening condition and the constraint condition from among candidate designs associated with the newness class.

In step S9-3, the candidate design search unit 106 reads the evaluation criterion from the evaluation criterion storing unit 113. Next, the candidate design search unit 106 obtains a difference in the formulations between the candidate design after the filtering and the existing design. Subsequently, the candidate design search unit 106 classifies the difference in the formulations based on the evaluation criterion. Then, the candidate design search unit 106 determines a newness class corresponding to the classification of the difference in the formulations based on the evaluation criterion.

In step S9-4, the candidate design search unit 106 determines whether the newness class determined in step S9-3 falls within a predetermined range. The predetermined range is indicated in the search range, and corresponds to a range of the newness class in which the number of candidate designs from which the proposed design is extracted is 1 or greater. In the present embodiment, the newness class in the predetermined range is A or B. In a case where the newness class falls within the predetermined range (YES), the candidate design search unit 106 acquires the next existing design, and returns the process to step S9-3. On the other hand, in a case where the newness class is outside the predetermined range (NO), the candidate design search unit 106 discards the determined candidate design, acquires the next candidate design from the filtered candidate designs, and returns the process to step S9-3. When it is determined that the newness class with respect to all of the existing designs falls within the predetermined range, the process advances to step S9-5.

In step S9-5, the candidate design search unit 106 adds the candidate design to the proposed design. The candidate design for which the process advances to step S9-5 has a newness class (A or B in this example) within the predetermined range with respect to all of the existing designs. Accordingly, it is possible to add only the candidate design that is not similar to the existing design to the proposed design.

In step S9-6, the candidate design search unit 106 determines whether the proposed design satisfies a search range. In the present embodiment, the search range is the number of proposed designs for each newness class. That is, the candidate design search unit 106 determines whether or not the number of candidate designs set in the search range is included in the proposed design.

In a case where the proposed design satisfies the search range (YES), the candidate design search unit 106 ends the proposed design extraction process. On the other hand, in a case where the proposed design does not satisfy the search range (NO), the candidate design search unit 106 advances the process to step S9-7.

In step S9-7, the candidate design search unit 106 adds the candidate design added to the proposed design in the step S9-5 to the existing design. Next, the candidate design search unit 106 acquires the next candidate design, and returns the process to step S9-3.

The candidate design search unit 106 repeats the processes of step S9-3 through step S9-7 until the next candidate design is no longer obtained. Accordingly, whether or not the candidate design is to be added to the proposed design is determined for all of the candidate designs associated with the newness class.

A description will be made by referring back to FIG. 4. In step S10, the result output unit 107 of the design evaluation device 10 receives the proposed design from the candidate design search unit 106. Next, the result output unit 107 generates evaluation result data based on the proposed design. Subsequently, the result output unit 107 transmits the generated evaluation result data to the user terminal 20.

The result output unit 107 includes the proposed design associated with the newness class in the evaluation result data. The result output unit 107 may include, in the evaluation result data, information related to a reference design or an experimented design indicating a material substance having a different type or a different formulation amount from the proposed design, for each proposed design.

In step S11, the result display unit 204 of the user terminal 20 receives the evaluation result data from the design evaluation device 10. Next, the result display unit 204 displays a result display screen for displaying the evaluation result on the display device 506. Then, the result display unit 204 displays the evaluation result indicated by the evaluation result data on the result display screen.

### <<Result Display Screen>>

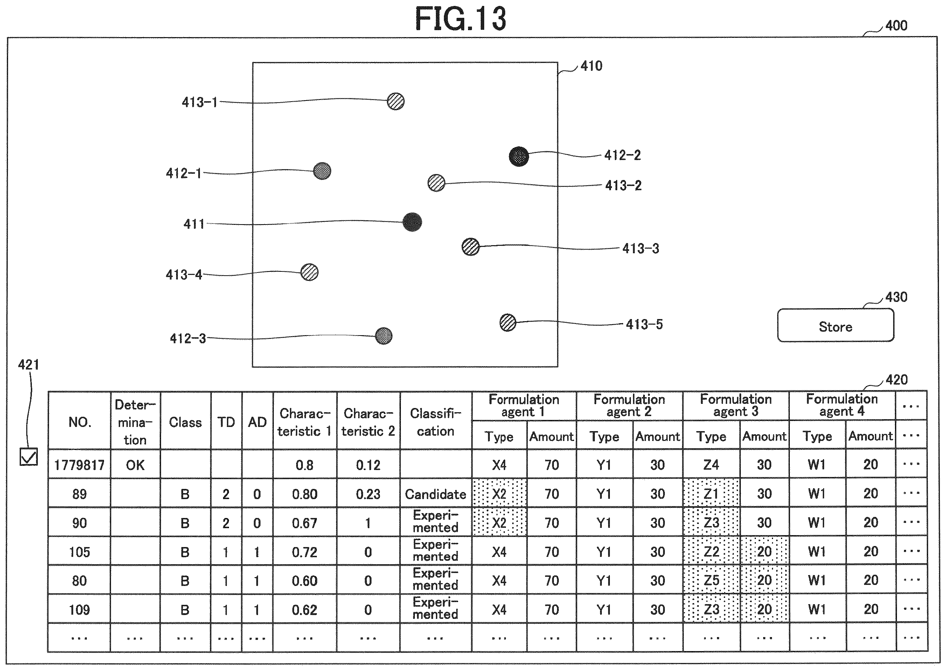

The result display screen in the present embodiment will be described with reference to FIG. 13. FIG. 13 is a diagram illustrating an example of the result display screen in the present embodiment.

As illustrated in FIG. 13, a result display screen 400 in this embodiment includes a design selection field 410, a design display field 420, and a store button 430.

The design selection field 410 displays a two-dimensional space in which a reference design 411 and proposed designs 412 and 413 are plotted. The design selection field 410 displays the proposed designs 412 and 413 in color, for example, according to the newness class. In the example illustrated in FIG. 13, the newness classes are identified by the line type and the coloring method in the display of the plots in the design selection field 410, and the proposed design 412 is the newness class A (challenging), while the proposed design 413 is the newness class B (robust).

The two-dimensional space is a plane obtained by dimensionally compressing the design space of the target substance in two dimensions. Any known method may be used for the dimensional compression method, and a method that enables easy understanding of the relationship among the designs may be adopted.

In the design display field 420, the proposed design and the existing design similar to the proposed design are displayed in a table format. The design display field 420 includes identification information (NO.) for identifying the design, a determination result (determination) indicating whether or not to propose, a newness class, a formulation type distance (TD), a formulation amount distance (AD), a predicted characteristic value (characteristic 1, characteristic 2), a classification of the design, and a formulation of the design (formulation agent 1 (type, amount) through formulation agent 4 (type, amount)). The number of predicted characteristic values and formulation agents matches the number of candidate designs and experimented designs.

A design in which a value is set for the determination is the candidate design, and a design in which no value is set for the determination is the existing design. The classification is a type of design (experimented design or candidate design) included in the existing design.

A predetermined number of existing designs in ascending order of the difference in formulations from the candidate design is included in each candidate design. In this case, the existing design represents the elements used as a material for the determination in an easily and visually recognizable form. In the present embodiment, the value of the existing design having a formulation different from that of the candidate design is colored. In the example illustrated in FIG. 13, the value in the design display field 420 having the formulation different from that of the candidate design is identified by a cell with a dot pattern.

The proposed designs 412 and 413 plotted in the design selection field 410 are displayed in a selectable manner. When one of the proposed designs 412 and 413 is selected from the design selection field 410, a row corresponding to the selected proposed design is highlighted in the design display field 420. In the example illustrated in FIG. 13, the selection display field 421 is displayed in each row of the design display field 420, and the selection display field 421 corresponding to a proposed design 412-2 selected in the design selection field 410 is displayed in a selected state.

When the user presses the store button 430, an evaluation result file in which the evaluation result is described is downloaded to the user terminal 20. The evaluation result file includes information related to the candidate design for which the determination is NG, in addition to the proposed design for which the determination is OK.

### <<Evaluation Result File>>

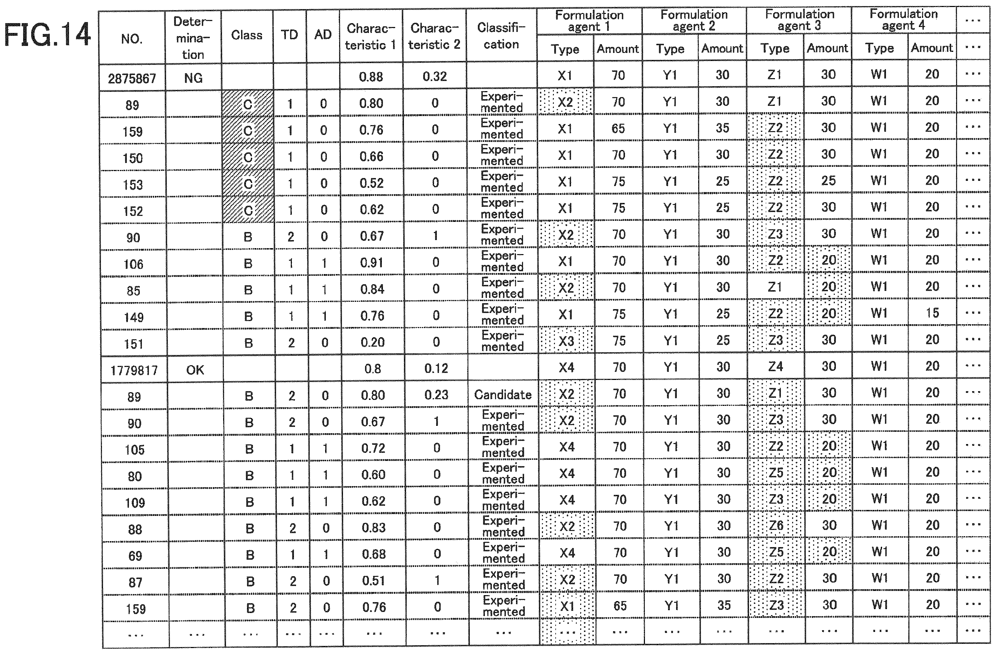

The evaluation result file in the present embodiment will be described with reference to FIG. 14. FIG. 14 is a diagram illustrating an example of an evaluation result file in the present embodiment.

As illustrated in FIG. 14, the evaluation result file in the present embodiment includes a NO., a determination, a class, a TD, an AD, characteristic 1, characteristic 2, a classification, and formulation agent 1 (type, amount) through formulation agent 4 (type, amount)), similar to the design display field 420 of the result display screen 400. However, the evaluation result file includes information related to candidate design for which the determination is NG.

### <<Effects of Embodiment>>

The design evaluation device 10 of the present embodiment determines a class indicating a degree of extrapolation of a candidate design of a target substance, based on the classification indicating a difference in formulations of material substances, and outputs the candidate design and the class in association with each other. Hence, according to the design evaluation device 10 of the present embodiment, it is possible to evaluate the degree of extrapolation of the candidate design of the target substance.

The design evaluation device 10 of the present embodiment determines a class preset for a combination of the classification based on the number of different types of material substances and the classification based on the number of different formulation amounts of material substances. The class is set with respect to the combination, in response to a user operation. Accordingly, the design evaluation device 10 of the present embodiment can output the degree of extrapolation according to the number of different types of material substances and the number of different formulation amounts of the material substances.

The design evaluation device 10 of the present embodiment determines the class for each of a plurality of candidate designs, and outputs a candidate design in which the class falls within a predetermined range. Hence, according to the design evaluation device 10 of the present embodiment, it is possible to propose the formulation of the target substance based on the evaluation result of the degree of extrapolation.

The design evaluation device 10 of the present embodiment evaluates the degree of extrapolation of each candidate design, based on the reference design selected by the user. Thus, the design evaluation device 10 of the present embodiment can propose a candidate design according to a direction of an experiment desired by the user.

The design evaluation device 10 of the present embodiment evaluates the degree of extrapolation of each candidate design, based on the evaluation criterion set by the user. Accordingly, the design evaluation device 10 of the present embodiment can provide a candidate design that is highly convincing to the user.

The design evaluation device 10 of the present embodiment further determines the class with respect to an experimented design in which the physical property of the target substance is known, and outputs a candidate design in which both the class with respect to the reference design and the class with respect to the experimented design fall within the predetermined range. Hence, according to the design evaluation device 10 of the present embodiment, it is possible to propose the formulation of the target substance based on the evaluation result of the degree of extrapolation with respect to the experimented design.

The user can determine a formulation to be experimented next, for example, based on the evaluation result obtained by the design support system 1 of the present embodiment. According to the design support system 1 of the present embodiment, because it is possible to obtain a candidate design in which the degree of extrapolation with respect to the reference design selected by the user satisfies the evaluation criterion set by the user, the user can obtain a candidate design according to the desired direction of the experiment, and can efficiently develop a new substance.

### [Supplement]

Each function of the embodiments described above can be implemented in one or a plurality of processing circuits. The "processing circuit" in the present specification includes a processor programmed to execute each function by software, such as a processor implemented by an electronic circuit, and a device, such as an application specific integrated circuit (ASIC), a digital signal processor (DSP), a field programmable gate array (FPGA), a conventional circuit module, or the like designed to perform each function described above.

Although the embodiments of the present invention are described above in detail, the present invention is not limited to these embodiments, and various variations and modifications can be made within the scope of the subject matter of the present invention recited in the claims.

This application claims priority to Japanese Patent Application No. 2022-207109 filed on December 23, 2022 before the Japan Patent Office, the entire contents of which are incorporated herein by reference.

### DESCRIPTION OF REFERENCE NUMERALS

1: Design support system
10: Design evaluation device
101: Evaluation criterion setting unit
102: Reference design determination unit
103: Candidate design determination unit
104: Class determination unit
105: Extraction condition setting unit
106: Candidate design search unit
107: Result output unit
111: Candidate design storing unit
112: Experimental result storing unit
113: Evaluation criterion storing unit
20: User terminal
201: Evaluation criterion input unit
202: Reference design input unit
203: Extraction condition input unit
204: Result display unit

## Claims

1. A design evaluation device comprising:
a reference design determination unit configured to determine a reference setting indicating a formulation of a target substance to be produced using two or more material substances;
a candidate design determination unit configured to determine a candidate design indicating the formulation that is different from the reference design;
a class determination unit configured to determine a class indicating a degree of extrapolation of the candidate design, based on a classification indicating a difference in the formulation between the candidate design and the reference design; and
a result output unit configured to output the candidate design and the class in association with each other.

2. The design evaluation device as claimed in claim 1, wherein the class is preset with respect to a combination of a number of different types of the material substances and a number of different formulation amounts of the material substances.

3. The design evaluation device as claimed in claim 2, wherein the class is set with respect to the combination, in response to a user operation.

4. The design evaluation device as claimed in any one of claims 1 to 3, wherein:
the class determination unit is configured to determine the class of each candidate design of a plurality of candidate designs, and
the result output unit is configured to output a candidate design in which the class falls within a predetermined range among the plurality of candidate designs.

5. The design evaluation device as claimed in claim 4, wherein:
the class determination unit is configured to further determine the class, based on a classification indicating a difference in the formulation between a candidate design of the plurality of candidate designs and an experimented design having a known physical property of the target substance for each candidate design among the plurality of candidate designs, and
the result output unit is configured to output the candidate design in which both the class with respect to the reference design and the class with respect to the experimented design fall within the range.

6. The design evaluation device as claimed in claim 5, wherein the result output unit is configured to output information related to the reference design or the experimented design indicating the material substance having a formulation type or amount different from the candidate design.

7. A design evaluation method by which a computer performs:
a procedure determining a reference design indicating a formulation of a target substance to be produced using two or more material substances;
a procedure determining a candidate design indicating the formulation that differs from the reference design;
a procedure determining a class indicating a degree of extrapolation of the candidate design, based on a classification indicating a difference in the formulation between the candidate design and the reference design; and
a procedure outputting the candidate design and the class in association with each other.

8. A program for causing a computer to execute:
a procedure determining a reference design indicating a formulation of a target substance to be produced using two or more material substances;
a procedure determining a candidate design indicating the formulation that differs from the reference design;
a procedure determining a class indicating a degree of extrapolation of the candidate design, based on a classification indicating a difference in the formulation between the candidate design and the reference design; and
a procedure outputting the candidate design and the class in association with each other.
